# EUROPEAN PATENT APPLICATION

(11) **EP 4 810 498 A1**
(43) Date of publication of application: **23.09.2026**
(21) Application number: 24890653.9
(22) Date of filing: 12.11.2024
(51) Int. Cl.: C07D 493/20, C07D 405/14, A61K 31/497, A61K 31/357, A61P 33/06

(54) **MEDICATION FOR TREATING SEVERE MALARIA AND SYNTHESIS METHOD THEREFOR**

(30) Priority: 13.11.2023 CN 202311498156
(71) Applicant: Institute of Chinese Materia Medica, China Academy of Chinese Medical Sciences, Dongzhimen Nei Dongcheng District Beijing 100700 (CN)
(72) Inventor: LI, Yujie, Beijing 100700 (CN); SUN, Peng, Beijing 100700 (CN); CHEN, Lina, Beijing 100700 (CN); SHEN, Shuo, Beijing 100700 (CN); LIU, Tuo, Beijing 100700 (CN); LI, Yingfei, Beijing 100700 (CN); WANG, Luqi, Beijing 100700 (CN); LIANG, Yan, Beijing 100700 (CN); LIU, Chengcheng, Beijing 100700 (CN); ZHANG, Shuaichen, Beijing 100700 (CN); MA, Jiahui, Beijing 100700 (CN); LI, Yu, Beijing 100700 (CN); LI, Chengteng, Beijing 100700 (CN)
(74) Representative: Winter, Brandl - Partnerschaft mbB
(86) International application number: PCT/CN2024/131516
(87) International publication number: WO 2025/103298

(57) **Abstract**

The present invention relates to a medication for treating severe malaria and a synthesis method therefor. The medication has a remarkable treatment effect on severe malaria (cerebral malaria), the medication can effectively eliminate plasmodium, reduce mortality, relieve nerve damage, and effectively reduce the malaria reinfection rate. The chemical structure of the medication is shown as a formula (I).

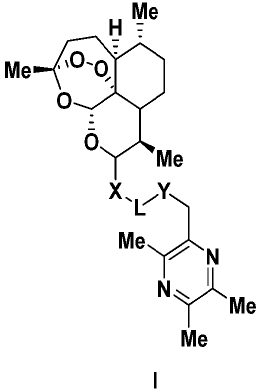

## Description

### TECHNICAL FIELD

The present invention belongs to the technical field of pharmaceuticals, and relates to a class of artemisinin/tetramethylpyrazine conjugates, and their pharmaceutically acceptable salts, hydrates, and optical isomers, as well as pharmaceutical compositions using the conjugates as active ingredients, and use thereof for treating severe malaria. In view of the pathological features of severe malaria and its often accompanied nervous system injury, the present invention designs and synthesizes a class of artemisinin/tetramethylpyrazine conjugates with novel structures by applying the combination principle of pharmaceutical chemistry, and the chemical structures of the conjugates are characterized and determined by 1NMR, 13NMR, and HRMS-ESI.

### BACKGROUND

Cerebral malaria (CM) is the most serious complication caused by infection of Plasmodium falciparum, which is the leading cause of death in children under 5 years old in Africa. It can lead to long-term cognitive and motor dysfunction in surviving children and thus becomes an urgent clinical problem in the field of malaria treatment.

74% of cerebral malaria cases occurred in African children under the age of five, with mortality rate of 15-20% (World Health Organization, 2016. de Miranda AS, 2010). Cerebral malaria presents with acute diffuse encephalitis, accompanied by various neurological symptoms clinically, such as coma, disturbance of consciousness, meningeal irritation signs, hemiplegia, convulsions, ataxia, and even death. Moreover, 10-17% of surviving children suffered from persistent neurological impairment, and cognitive and motor dysfunction (Birbeck GL, 2010; de Miranda AS, 2010), where the motor ability, language ability, and social communication ability of the children patient under 5 years old might be delayed for 6 months or more, and the delay would be more significant for those with obvious abnormalities in MRI.

According to the theory of "mechanical obstruction" of cerebral malaria, the adhesion and blockage of parasitized red blood cells (pRBCs) in cerebral microvessels is the initial factor leading to the occurrence of cerebral malaria, and a large number of pRBCs are "retained" in microvessels, resulting in a decrease in blood flow perfusion and hypoxia and ischemia of brain tissue, which is the basis of cerebral malaria onset and cerebral malaria-induced secondary nerve injury.

The local embolic changes in cerebral microvessels, microcirculation disturbance, olfactory bulb bleeding, changes in blood rheology, and platelet function abnormalities that occur during the process of cerebral malaria meet the diagnostic criteria of "blood stasis syndrome" in traditional Chinese medicine. Its neurological symptoms such as high fever, irritability, coma, delirium, and cerebral hemorrhage are all secondary lesions based on "blood stasis".

Artesunate (AS), also known as dihydroartemisinin-12-α-succinate, has good solubility and bioavailability. It is water-soluble, can penetrate the blood-brain barrier, and can be administered through various routes such as intravenous injection, intramuscular injection, oral administration, and rectal administration. Artesunate can maintain at a high concentration in the nervous system and has the characteristics of high efficacy and low toxicity. Intravenous injection of artesunate is recommended as the preferred treatment plan for cerebral malaria by WHO in the *Management of Severe Malaria: A Practical Handbook.* As a current first-line treatment drug, artesunate has a market share of 90% or more in the global severe malaria market. Increasing evidence shows that artesunate has multiple pharmacological activities such as anti-inflammatory, antioxidant, blood-brain barrier protection, immunoregulation, antibacterial, and anti-tumor.

Artesunate has the following problems in the clinical treatment of cerebral malaria. ① According to the *Management of Severe Malaria: A Practical Handbook* (the Third Edition) published by WHO, due to the reduction in sensitivity of Plasmodium falciparum to artemisinin-based drugs, the recommended dosage of artesunate for treating cerebral malaria has been greatly increased, the first-day treatment dose for adults has increased from 120 mg to 432 mg, and the full course treatment dose in 7 days has increased from 480 mg to 1296 mg. Therefore, the treatment cost, the risk of drug resistance for monotherapy application, and the risk of side effects such as hemolysis have continued to increase. ② Artesunate has poor efficacy in late-stage cerebral malaria, which has shown signs of inflammatory response and blood-brain barrier disruption in the host. ③ After artesunate killed the plasmodia, dead bodies of the parasites still block in local brain cerebral microvessels, resulting in a failure in effectively alleviating the state of ischemia and hypoxia. ④ Artesunate cannot effectively intervene in the persistent neurological impairment and cognitive and motor dysfunction of surviving children with cerebral malaria. ⑤ In clinical practice, intravenous injection of artesunate is used to treat severe malaria such as cerebral malaria in the areas with high incidence of cerebral malaria in Africa, and the extremely backward medical conditions have largely limited the timely and effective clinical application of artesunate. ⑥ Artesunate is administered intravenously, which is poor in compliance for the high-risk population of infants and young children under 5 years old.

Tetramethylpyrazine (TMP) is considered a characteristic alkaloid of Chuanxiong Rhizoma, and its role in the acute injury stage of ischemic stroke has been confirmed in multiple studies. For example, it has a protective effect on brain injury caused by reperfusion after ischemic stroke, can effectively ameliorate behavioral and learning memory retention disorders after brain injury, and can also alleviate the deterioration of stroke condition and thus reduce mortality or disability rates. It has been reported that a tetramethylpyrazine nasal spray can effectively increase the concentration of Chuanxiong Rhizoma in brain tissue and give full play to its therapeutic effect.

Previous studies have confirmed that the combination of artesunate and tetramethylpyrazine administered intranasally has a definite therapeutic effect on cerebral malaria, which can significantly reduce the mortality rate and parasitemia level of mice with cerebral malaria, and effectively alleviate nerve injury in mice; and its effect is related to increasing cerebral blood flow perfusion and improving blood oxygen supply to the nerves. One of its key action links is to determine through the mutation of a nitrosylation site (cysteine C263) that it affects the balance of a NO-related redox system by regulating the nitrosylation of pyruvate dehydrogenase E1 subunit beta (PDHB).

However, the chemical compatibility between artesunate and tetramethylpyrazine is poor, which poses difficulties for the development of compound antimalarial preparations and affects their pharmacological properties. In terms of chemical structure, artesunate is a monoester formed by dihydroartemisinin and succinic acid, with a free carboxyl group in the molecule. In order to improve the water solubility of the compound, its sodium salt, i.e., sodium artesunate, is used as an injection in clinic, which is alkaline. Tetramethylpyrazine is an oily compound that is difficult to dissolve in water. In order to improve its water solubility and stability as well, its hydrochloride salt, i.e., ligustrazine hydrochloride, is clinically used, which has strong acidity. When tetramethylpyrazine and artesunate are co-dissolved in an aqueous or organic phase solvent system, the ester bond of artesunate is rapidly hydrolyzed, resulting in a rapid decrease in content. When co-dissolved in an alkaline aqueous phase solvent, they will undergo a violent acid-base neutralization reaction, resulting in the immediate precipitation of artesunate and the production of insoluble oily tetramethylpyrazine, which cannot form a uniform and stable preparation. In order to solve this problem, various buffer systems and the use of nanomedicine technology have been tried, but none of them can effectively solve this problem.

The present invention obtains a novel compound by chemically combining artesunate and tetramethylpyrazine, which solves the problems existing in a compound preparation of artesunate and tetramethylpyrazine and retains the efficacy advantages of their combined use.

### SUMMARY

It is an object of the present invention to provide a class of artemisinin/tetramethylpyrazine conjugates, and their pharmaceutically acceptable salts, hydrates, and prodrugs. These compounds can be used for preparing novel drugs for the treatment of severe malaria.

To this end, the present invention provides a drug comprising Formula I or a pharmaceutically acceptable salt thereof: 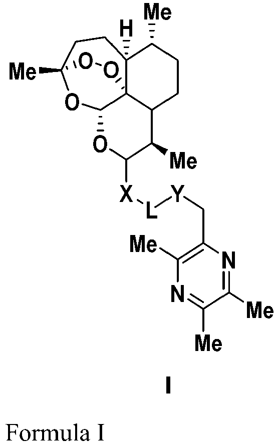
wherein X and Y are each independently selected from the group consisting of CH₂, O, S, and NH; and
L is a linker selected from the group consisting of: 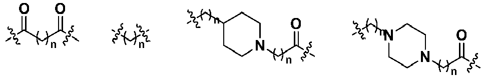 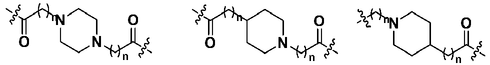
wherein n is an integer from 0 to 6.

Preferably, the drug or the pharmaceutically acceptable salt thereof according to the present invention comprises a structure as follows: 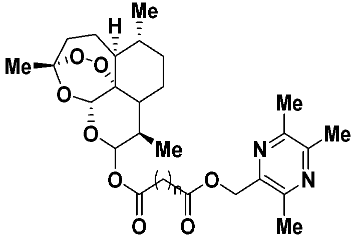 wherein n is an integer from 0 to 6.

Most preferably, the drug or the pharmaceutically acceptable salt thereof according to the present invention comprises a structure as follows:

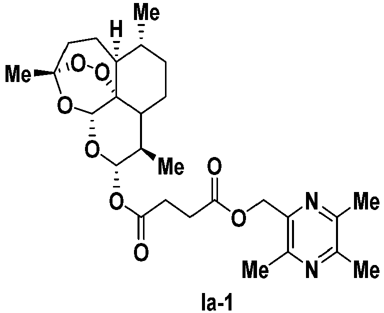

The most preferred drug of the present invention is a completely new compound, which is referred to as Artesunazine in this description, also called compound Ia-1, and also known as artemisinin/tetramethylpyrazine conjugate, with a chemical name of (3R, 5aS, 6R, 9R, 10R, 12R, 12aR)-3, 6, 9-trimethyldecahydro-12h-3, 12-epoxy [1, 2] dioxopyran [4, 3-i] isobenzopyran-10-yl ((3, 5, 6-trimethylpyrazine-2-yl) methyl) succinate. The nuclear magnetic resonance and high-resolution mass spectrometry data of the compound are as follows: ¹H NMR (600 MHz, cdcl₃) δ 5.79 (d, J = 9.9 Hz, 1H), 5.43 (s, 1H), 5.21 (s, 2H), 2.82 - 2.73 (m, 3H), 2.73 - 2.66 (m, 1H), 2.54 - 2.49 (m, 9H), 2.39 (d, J = 13.5 Hz, 1H), 2.03 (d, J = 15.0 Hz, 2H), 1.89 (s, 1H), 1.80 - 1.70 (m, 2H), 1.63-1.60 (m, 1H), 1.50-1.47 (m, 1H), 1.43 (s, 3H), 1.41 - 1.23 (m, 4H), 1.03 (t, J = 10.6 Hz, 1H), 0.97 (d, J = 6.2 Hz, 3H), 0.83 (d, J = 7.1 Hz, 3H). ¹³C NMR (151 MHz, cdcl₃) δ 171.80, 171.00, 151.34, 149.08, 148.98, 144.52, 104.45, 92.16, 91.47, 80.08, 65.34, 51.52, 45.19, 37.24, 36.18, 34.06, 31.76, 29.13, 28.69, 25.94, 24.55, 21.96, 21.69, 21.45, 20.49, 20.21, 12.01. HRMS (ESI) calcd for [C₂₇H₃₈N₂O₈+H]+ 519.2628, found 519.2487. 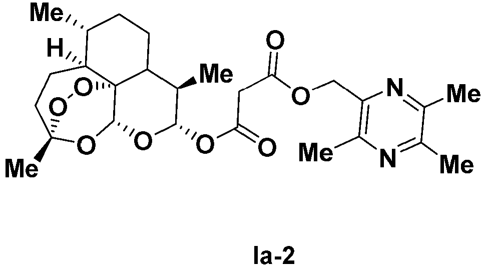 It is called compound Ia-2, with a chemical name of (3R, 5aS, 6R, 9R, 10R, 12R, 12aR)-3, 6, 9-trimethyldecahydro-12h-3, 12-epoxy [1, 2] dioxopyran [4, 3-i] isobenzopyran-10-yl ((3, 5, 6-trimethylpyrazine-2-yl) methyl) malonate. The nuclear magnetic resonance and high-resolution mass spectrometry data of the compound are as follows: ¹H NMR (600 MHz, cdcl₃) δ 5.60 (d, J = 9.7 Hz, 1H), 5.35 (s, 1H), 5.21 (s, 2H), 3.40 (s, 2H), 2.82 - 2.73 (m, 3H), 2.73 - 2.66 (m, 1H), 2.54 - 2.49 (m, 5H), 2.39 (d, J = 13.5 Hz, 1H), 2.03 (d, J = 14.0 Hz, 2H), 1.89 (s, 1H), 1.80 - 1.70 (m, 2H), 1.63-1.60 (m, 1H), 1.50-1.47 (m, 1H), 1.43 (s, 3H), 1.41 - 1.23 (m, 3H), 1.03 (t, J = 10.6 Hz, 1H), 0.97 (d, J = 6.2 Hz, 3H), 0.83 (d, J = 7.1 Hz, 3H). ¹³C NMR (151 MHz, cdcl₃) δ 171.60, 171.20, 151.24, 149.32, 148.87, 144.50, 104.32, 92.18, 91.47, 80.08, 65.34, 51.52,47.32, 45.19, 37.24, 36.18, 34.06, 28.52, 25.84, 24.35, 21.91, 21.70, 21.42, 20.42, 20.12, 12.07. HRMS (ESI) calcd for [C₂₆H₃₆N₂O₈+H]+ 505.2550, found 505.2547. 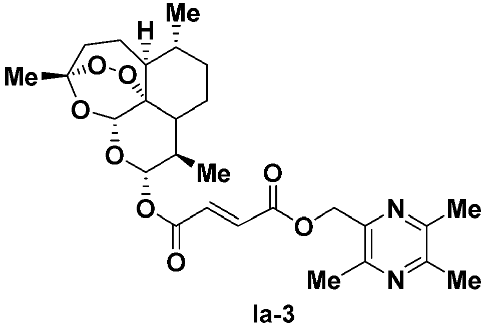 It is called compound Ia-3, with a chemical name of (3R, 5aS, 6R, 9R, 10R, 12R, 12aR)-3, 6, 9-trimethyldecahydro-12h-3, 12-epoxy [1, 2] dioxopyran [4, 3-i] isobenzopyran-10-yl ((3, 5, 6-trimethylpyrazine-2-yl) methyl) maleate. The nuclear magnetic resonance and high-resolution mass spectrometry data of the compound are as follows: 1H NMR (600 MHz, cdcl3) δ 6.98 (d, J = 10.2 Hz, 1H), 5.79 (d, J = 9.9 Hz, 1H), 5.43 (s, 1H), 5.33(d, J = 10.7 Hz, 1H), 5.21 (s, 2H), 2.54 - 2.49 (m, 9H), 2.39 (d, J = 13.5 Hz, 1H), 2.03 (d, J = 13.0 Hz, 2H), 1.89 (s, 1H), 1.80 - 1.70 (m, 2H), 1.63-1.60 (m, 1H), 1.50-1.47 (m, 1H), 1.43 (s, 3H), 1.41 - 1.23 (m, 3H), 1.03 (t, J = 10.6 Hz, 1H), 0.97 (d, J = 6.2 Hz, 3H), 0.83 (d, J = 7.1 Hz, 3H). ¹³C NMR (151 MHz, cdcl3) δ 164.37, 163.50, 151.64, 149.52, 147.61, 144.01, 134.06, 133.71, 104.50, 92.86, 91.53, 80.01, 65.85, 51.49, 45.16, 37.24, 36.16, 34.03, 31.72, 29.68, 25.89, 24.54, 21.95, 21.69, 21.44, 20.48, 20.19, 12.06. HRMS (ESI) calcd for [C₂₇H₃₆N₂O₈+H]+ 517.2550, found 517.2550. 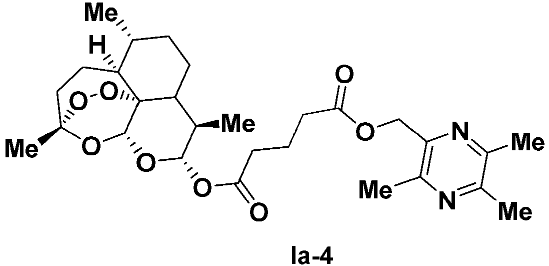 It is called compound Ia-4, with a chemical name of (3R, 5aS, 6R, 9R, 10R, 12R, 12aR)-3, 6, 9-trimethyldecahydro-12h-3, 12-epoxy [1, 2] dioxopyran [4, 3-i] isobenzopyran-10-yl ((3, 5, 6-trimethylpyrazine-2-yl) methyl) glutarate. The nuclear magnetic resonance and high-resolution mass spectrometry data of the compound are as follows: H NMR (600 MHz, cdcl₃) δ 5.78 (d, J = 9.9 Hz, 1H), 5.45 (s, 1H), 5.21 (s, 2H), 2.82 - 2.73 (m, 3H), 2.73 - 2.66 (m, 1H), 2.53 - 2.47 (m, 9H), 2.39 (d, J = 13.7 Hz, 1H), 2.02 (d, J = 14.5 Hz, 2H), 1.89 (s, 1H), 1.80 - 1.70 (m, 2H), 1.63-1.60 (m, 1H), 1.50-1.47 (m, 3H), 1.41 (s, 3H), 1.41 - 1.23 (m, 3H), 1.03 (t, J = 10.6 Hz, 1H), 0.97 (d, J = 6.0 Hz, 3H), 0.84 (d, J = 7.0 Hz, 3H). ¹³C NMR (150 MHz, cdcl₃) δ 172.35, 171.53, 151.05, 148.90, 148.65, 104.23, 91.75, 91.27, 79.92, 64.67, 51.38, 45.06, 37.04, 36.06, 33.94, 32.97, 32.81, 31.58, 29.54, 25.74, 24.44, 21.79, 21.43, 21.24, 20.24, 20.10, 19.69, 11.98. HRMS (ESI) calcd for [C₂₈H₄₀N₂O₈+H]+ 533.2863, found 533.2851. 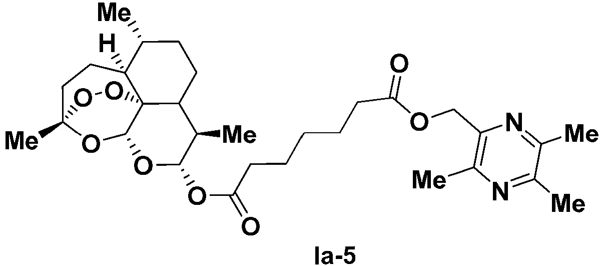 It is called compound Ia-5, with a chemical name of (3R, 5aS, 6R, 9R, 10R, 12R, 12aR)-3, 6, 9-trimethyldecahydro-12h-3, 12-epoxy [1, 2] dioxopyran [4, 3-i] isobenzopyran-10-yl ((3, 5, 6-trimethylpyrazine-2-yl) methyl) pimelate. The nuclear magnetic resonance and high-resolution mass spectrometry data of the compound are as follows: ¹H NMR (600 MHz, cdcl₃) δ 5.79 (d, J = 9.8 Hz, 1H), 5.44 (s, 1H), 5.19 (s, 2H), 2.82 - 2.73 (m, 3H), 2.73 - 2.66 (m, 1H), 2.54 - 2.49 (m, 9H), 2.40 (d, J = 14.5 Hz, 1H), 2.04 (d, J = 15.3 Hz, 2H), 1.89 (s, 1H), 1.80 - 1.70 (m, 2H), 1.63-1.60 (m, 1H), 1.51-1.44 (m, 1H), 1.41 (s, 3H), 1.41 - 1.36 (m, 3H),1.31 - 1.24 (m, 4H),10.5-1.02 (m, 3H), 0.97 (d, J = 6.2 Hz, 3H), 0.83 (d, J = 7.1 Hz, 3H). ¹³C NMR (151 MHz, cdcl₃) δ 172.92, 172.01, 151.03, 148.79, 148.77, 144.72, 104.22, 91.58, 91.30, 79.94, 64.75, 51.43, 45.11, 37.08, 36.09, 33.98, 33.87, 33.68, 31.66, 28.36, 25.81, 24.47, 24.14, 21.84, 21.54, 21.32, 20.91, 20.36, 20.13, 12.02. HRMS (ESI) calcd for [C₃₀H₄₄N₂O₈+H]+ 561.3175, found 561.3181. 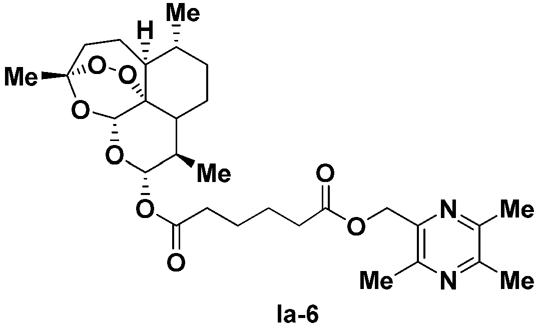 It is called compound Ia-6, with a chemical name of (3R, 5aS, 6R, 9R, 10R, 12R, 12aR)-3, 6, 9-trimethyldecahydro-12h-3, 12-epoxy [1, 2] dioxopyran [4, 3-i] isobenzopyran-10-yl ((3, 5, 6-trimethylpyrazine-2-yl) methyl) adipate. The nuclear magnetic resonance and high-resolution mass spectrometry data of the compound are as follows: ¹H NMR (600 MHz, cdcl₃) δ 5.79 (d, J = 9.5 Hz, 1H), 5.44 (s, 1H), 5.19 (s, 2H), 2.82 - 2.73 (m, 3H), 2.73 - 2.66 (m, 1H), 2.52 - 2.50 (m, 9H), 2.42 (d, J = 14.0 Hz, 1H), 2.04 (d, J = 14.0 Hz, 2H), 1.89 (s, 1H), 1.80 - 1.70 (m, 2H), 1.63-1.60 (m, 1H), 1.50-1.47 (m, 1H), 1.43 (s, 3H), 1.41 - 1.27 (m, 3H),1.27 - 1.23 (m, 4H) 1.03 (t, J = 10.6 Hz, 1H), 0.97 (d, J = 6.2 Hz, 3H), 0.83 (d, J = 7.5 Hz, 3H). ¹³C NMR (151 MHz, cdcl₃) δ 172.66, 171.76, 151.02, 149.36, 148.80, 144.66, 104.21, 91.64, 91.29, 79.92, 64.75, 51.42, 45.10, 37.07, 36.08, 33.97, 33.50, 31.63, 29.56, 25.79, 24.46, 24.16, 23.90, 21.83, 21.52, 21.31, 20.34, 20.11, 12.01. HRMS (ESI) calcd for [C₂₉H₄₂N₂O₈+H]+ 547.3019, found 547.3007. 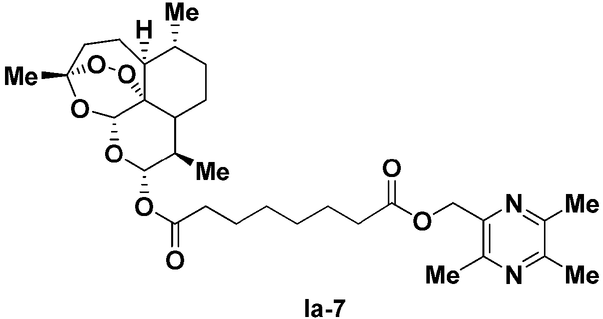 It is called compound Ia-7, with a chemical name of (3R, 5aS, 6R, 9R, 10R, 12R, 12aR)-3, 6, 9-trimethyldecahydro-12h-3, 12-epoxy [1, 2] dioxopyran [4, 3-i] isobenzopyran-10-yl ((3, 5, 6-trimethylpyrazine-2-yl) methyl) suberate. The nuclear magnetic resonance and high-resolution mass spectrometry data of the compound are as follows: ¹H NMR (600 MHz, cdcl₃) δ 5.79 (d, J = 9.0 Hz, 1H), 5.44 (s, 1H), 5.19 (s, 2H), 2.82 - 2.73 (m, 3H), 2.73 - 2.66 (m, 1H), 2.53 - 2.42 (m, 9H), 2.39 (d, J = 13.5 Hz, 1H), 2.03 (d, J = 15.0 Hz, 2H), 1.89 (s, 1H), 1.80 - 1.70 (m, 2H), 1.63-1.60 (m, 5H), 1.50-1.47 (m, 1H), 1.43 (s, 3H), 1.41 - 1.23 (m, 7H), 1.03 (t, J = 10.6 Hz, 1H), 0.97 (d, J = 6.2 Hz, 3H), 0.83 (d, J = 7.1 Hz, 3H). ¹³C NMR (151 MHz, cdcl₃) δ 173.16, 172.24, 151.10, 149.42, 148.84, 144.78, 91.60, 91.36, 80.01, 64.80, 51.47, 45.16, 37.14, 36.13, 34.07, 31.71, 28.64, 28.59, 25.87, 24.64, 24.50, 24.35, 21.90, 21.59, 21.37, 21.28, 21.17, 20.40, 20.17, 19.29, 12.07. HRMS (ESI) calcd for [C₃₁H₄₆N₂O₈+H]+ 575.3332, found 575.3342. 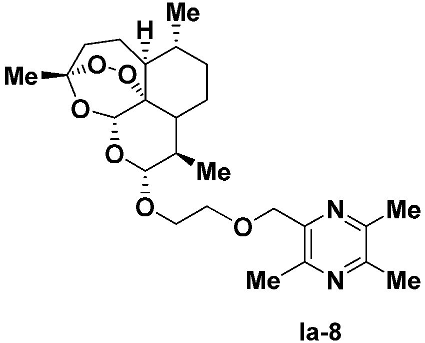 It is called compound Ia-8, with a chemical name of 2, 3, 5-trimethyl-6-[(2-(((3R, 5aS, 6R, 9R, 12R, 12aR)-3, 6, 9-trimethylbicyclo [3.2.1] heptane-12H-3, 12-epoxide [1, 2] dioxacycloheptane [4, 3-i] isochromene-10-yl) oxy) ethoxy) methyl] pyrazine. The nuclear magnetic resonance and high-resolution mass spectrometry data of the compound are as follows: ¹H NMR (600 MHz, cdcl₃) δ 5.79 (d, J = 9.1 Hz, 1H), 5.43 (s, 1H), 5.21 (s, 2H), 3.72 - 3.54 (m, 4H), 2.54 - 2.49 (m, 9H), 2.39 (d, J = 14.5 Hz, 1H), 2.03 (d, J = 15.0 Hz, 2H), 1.89 (s, 1H), 1.80 - 1.70 (m, 2H), 1.63-1.60 (m, 1H), 1.50-1.47 (m, 1H), 1.43 (s, 3H), 1.41 - 1.23 (m, 3H), 1.03 (t, J = 10.6 Hz, 1H), 0.97 (d, J = 6.2 Hz, 3H), 0.83 (d, J = 7.1 Hz, 3H). ¹³C NMR (150 MHz, cdcl₃) δ 171.80, 171.00, 151.34, 149.08, 148.98, 144.52, 104.45, 92.16, 91.47, 80.08,70.1,70.34, 65.34, 51.52, 45.19, 37.24, 36.18, 25.94, 24.55, 21.96, 21.69, 21.45, 20.49, 20.21, 12.01. HRMS (ESI) calcd for [C₂₅H₃₈N₂O₈+H]+ 463.2808, found 463.2810. 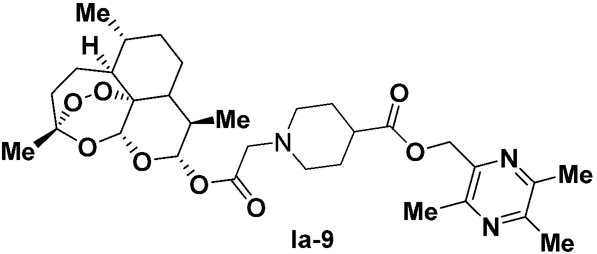 It is called compound Ia-9, with a chemical name of (3, 5, 6-trimethylpyrazine-2-yl) methyl 1-(2-oxy-2-(((3R, 5aS, 6R, 9R, 10S, 12R, 12aR)-3, 6, 9-trimethyldecahydro-12H-3, 12-epoxy [1, 2] dioxacycloheptane-10-yl) oxy) ethyl) piperidine-4-formate. The nuclear magnetic resonance and high-resolution mass spectrometry data of the compound are as follows: ¹H NMR (600 MHz, cdcl₃) δ 5.79 (d, J = 9.7 Hz, 1H), 5.43 (s, 1H), 5.21 (s, 2H),3.32 (s, 2H), 2.54 - 2.49 (m, 13H), 2.45 - 2.37 (m, 2H), 2.03 (d, J = 15.0 Hz, 2H), 1.97-1.89 (m, 5H), 1.80 - 1.70 (m, 2H), 1.63-1.60 (m, 1H), 1.50-1.47 (m, 1H), 1.43 (s, 3H), 1.41 - 1.23 (m, 3H), 1.03 (t, J = 10.6 Hz, 1H), 0.97 (d, J = 6.2 Hz, 3H), 0.83 (d, J = 7.1 Hz, 3H). ¹³C NMR (150 MHz, cdcl₃) δ 171.80, 171.00, 151.34, 149.08, 148.98, 144.52, 104.45, 92.16, 91.47, 80.08, 65.34, 57.44, 51.52, 45.19, 37.24, 36.18, 34.06, 31.76, 47.44, 47.02, 41.2, 29.21, 28.37, 25.94, 24.55, 21.96, 21.69, 21.45, 20.49, 20.21, 12.01.HRMS (ESI) calcd for [C₃₁H₄₅N₃O₈+H]+ 588.3285, found 588.3287. 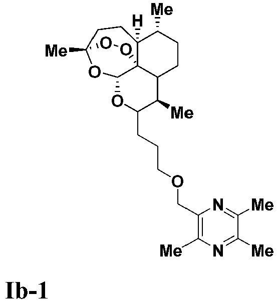 It is called compound Ib-1, with a chemical name of (3R, 5aS, 6R, 9R, 10R, 12R, 12aR)-3, 6, 9-trimethyldecahydro-12h-3, 12-epoxy-1, 2-dioxopyran [4, 3-i] isobenzopyran-10-yl ((3, 5, 6-trimethylpyrazine-2-yl) methyl) ether. The nuclear magnetic resonance and high-resolution mass spectrometry data of the compound are as follows: ¹H NMR (600 MHz, CDCl₃)δ 5.30 (s, 1H), 4.63 (s, 2H), 4.21-4.15 (m, 1H), 3.64-3.60 (m, 2H), 2.71-2.62 (m, 4H), 2.57-2.54 (m, 3H), 2.34-2.28 (m, 1H), 2.06-2.00 (m, 1H), 1.96-1.81 (m, 2H), 1.78-1.77 (m, 1H), 1.68-1.33 (m, 7H), 1.32-1.25 (m, 7 H), 0.97 (d, 3H), 0.86 (d, 3H).¹³C NMR (151 MHz, cdcl₃) δ149.71, 148.21, 147.62, 147.45, 121.28, 96.44, 89.23, 81.87, 72.72, 71.37, 51.77, 43.58, 37.01, 36.48, 32.93, 29.72, 29.51, 26.03, 25.12, 20.27, 19.26, 19.12, 19.01, 18.86. HRMS (ESI) calcd for [C₂₆H₄₀N₂O₈+H]+ 461.2915, found 461.2927.

The present invention further provides the pharmaceutically acceptable salt as described in the present invention, wherein the pharmaceutically acceptable salt is an addition salt formed by the drug according to the present invention and an acid selected from the group consisting of hydrochloric acid, hydrobromic acid, sulfuric acid, phosphoric acid, methanesulfonic acid, ethanesulfonic acid, p-toluenesulfonic acid, benzenesulfonic acid, naphthalene disulfonic acid, acetic acid, propionic acid, lactic acid, trifluoroacetic acid, maleic acid, citric acid, fumaric acid, oxalic acid, tartaric acid, benzoic acid, and the like; and hydrochloric acid, hydrobromic acid, sulfuric acid, lactic acid, pyruvic acid, acetic acid, trifluoroacetic acid, maleic acid, benzenesulfonic acid, and succinic acid.

The present invention further provides a pharmaceutical composition, including the drug or the pharmaceutically acceptable salt thereof according to the present invention. The pharmaceutical composition is in the form of a pharmaceutical preparation and further comprises pharmaceutically acceptable excipients as required for the preparation. The pharmaceutical composition according to the present invention is selected from any pharmaceutical dosage forms for administration, such as a nasal preparation, an injection, or an oral preparation.

The present invention further provides a preparation method of the drug or the pharmaceutically acceptable salt thereof according to the present invention.

The preparation method of the preferred drug according to the present invention includes following steps: 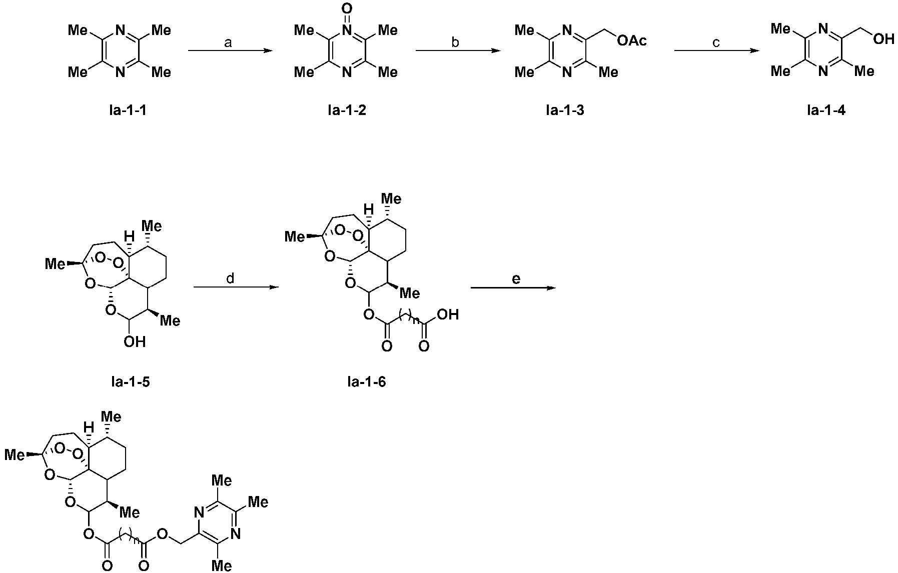 or 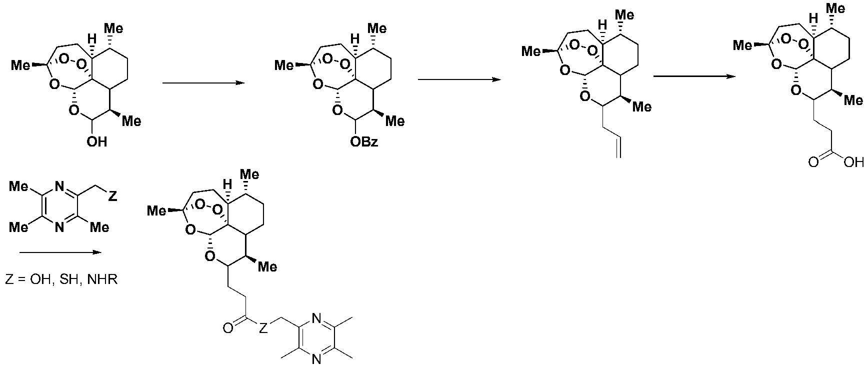 or 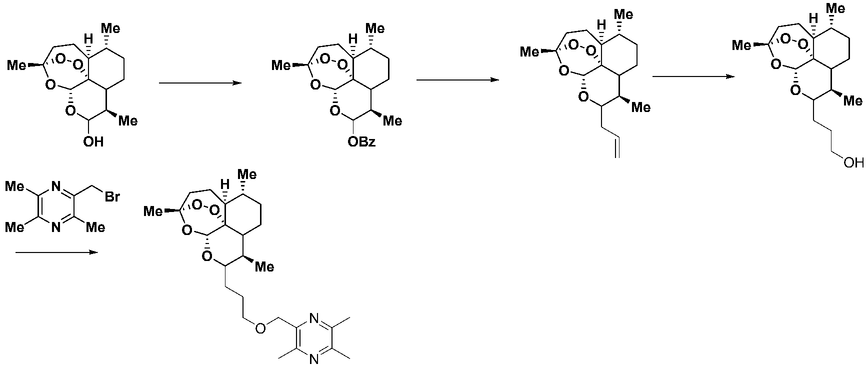 specifically,
step a: enabling a compound Ia-1-1 to react with hydrogen peroxide in presence of glacial acetic acid to obtain a compound Ia-1-2;
step b: enabling the compound Ia-1-2 to react with acetic anhydride to obtain a compound Ia-1-3;
step c: enabling the compound Ia-1-3 to undergo a hydrolysis reaction in presence of sodium hydroxide to obtain a compound Ia-1-4;
step d: enabling a compound Ia-1-5 to react with succinic anhydride to obtain a compound Ia-1-6; and
step e: enabling the compounds Ia-1-6 and Ia-1-4 to undergo a condensation reaction in presence of 1-ethyl-(3-dimethylaminopropyl) carbodiimide hydrochloride and 1-hydroxybenzotriazole to obtain the preferred drug of the present invention.

The drug can also be synthesized according to the steps outlined in the general solution, which includes different orders of synthesizing intermediates. The raw materials can be purchased commercially or prepared using known methods reported in documents.

The present invention further provides use of the drug or the pharmaceutically acceptable salt thereof according to the present invention in the preparation of a drug for treating or preventing severe malaria.

The drug or the pharmaceutically acceptable salt thereof according to the present invention further includes its solvates such as hydrates, optical isomers such as racemates or their isomers, as well as polymorphs such as different crystalline forms produced by crystallization from different solvents.

The compounds or their pharmaceutically acceptable salts, hydrates, or prodrugs involved in the present invention can be used as the sole antimalarial drug alone, or in combination with already marketed antimalarial drugs for the treatment and prevention of severe malaria.

The present invention solves the problems in the prior art and produces beneficial effects, mainly manifested in that:
1. By means of combination, the core chemical structures of the two chemical substances are preserved, resulting in completely new Artesunazine, (i.e., the compounds of the present invention), which improves the stability of the original artemisinin-tetramethylpyrazine compound in the same solvent system, thus providing a good foundation for the development of novel antimalarial drugs.
2. The Artesunazine provided by the present invention has a significant inhibitory effect on the proliferation of Plasmodium falciparum 3D7 within a nanomolar concentration range, which has an efficacy intensity comparable to that of the first-line drug artesunate for cerebral malaria recommended by WHO.
3. The Artesunazine provided by the present invention has outstanding protective effects on mouse cerebral malaria models, and is superior to artesunate in improving mouse survival rates and neurobehavioral scores, inhibiting recurrence, and the like.

### BRIEF DESCRIPTION OF THE DRAWINGS

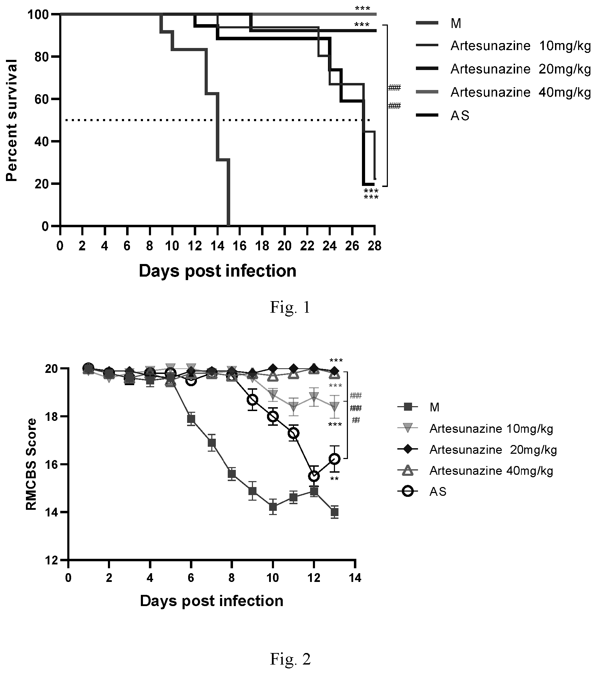

Fig. 1 shows the effects of drugs on survival curves of mice with cerebral malaria.
   ***p<0.001 indicates the statistical difference compared with a Model group; ###p<0.001 indicates the statistical difference compared with an AS group.
Fig. 2 shows the effects of drugs on rapid mouse coma behavior scale (RMCBS) scores of cerebral malaria model mice.
   * represents comparison with the M group, # represents comparison with the AS group, *** and ### represent significance P<0.001, ** and ## represent significance P<0.01.

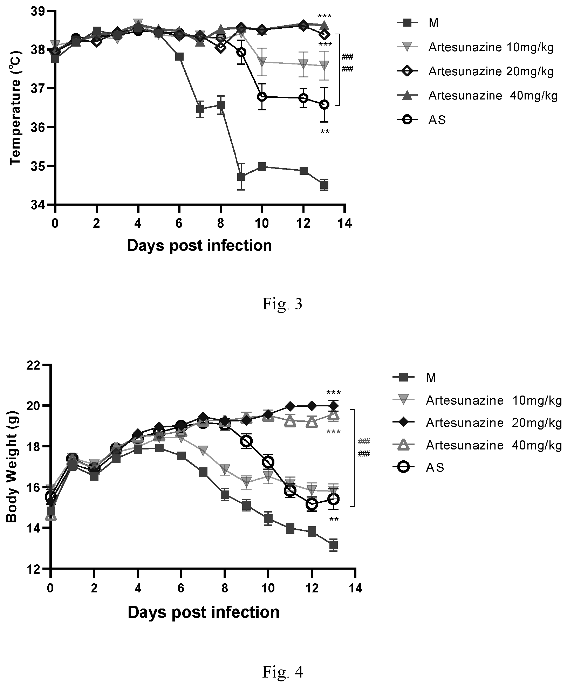

Fig. 3 shows the effects of drugs on temperature of cerebral malaria model mice.
   * represents comparison with the M group, # represents comparison with the AS group, *** and ### represent significance P<0.001, ** and ## represent significance P<0.01.
Fig. 4 shows the effects of drugs on body weight of cerebral malaria model mice.
   * represents comparison with the M group, # represents comparison with the AS group, *** and ### represent significance P<0.001, ** and ## represent significance P<0.01.
Fig. 5 shows the inhibitory effects of Artesunazine and AS on the growth of a Plasmodium falciparum standard strain 3D7.
Fig. 6 shows changes in contents of Artesunazine and artesunate in a stability experiment (the drug content was 100% on day 0).
Fig. 7 shows changes in a content of ligustrazine hydrochloride in the stability experiment (the drug content was 100% on day 0).

### DETAILED DESCRIPTION OF THE EMBODIMENTS

The embodiments and preparation examples provided below further illustrate and exemplify the compounds provided by the present invention and their preparation methods. It should be understood that the following embodiments and preparation examples do not limit the scope of the present invention in any way. Without further elaboration, it is believed that those skilled in the art can make maximum use of the present invention with the help of the foregoing descriptions. Therefore, the embodiments provided below are only intended to further illustrate the present invention, and are not meant to limit the scope of the present invention in any way.

The raw materials can be available commercially, or prepared either by known methods, or according to the methods described herein.

The structures of compounds were determined by nuclear magnetic resonance (¹H-NMR, ¹³C-NMR) and high-resolution mass spectrometry (HRMS-MS). H-NMR spectroscopy was measured by an AVANCE-600MHz nuclear magnetic resonance spectrometer, with deuterated chloroform (CDCl₃) or deuterated dimethyl sulfoxide (DMSO-d₆) determined to be a solvent, and tetramethylsilane (TMS) used as an internal standard. The mass spectrum was determined by Agilent 1100LC/MSD. 200-300 mesh silica gel (produced by Qingdao Marine Chemical Plant) was employed for column chromatography.

### Embodiment 1. Preparation of compound Ia-1:

(3R, 5aS, 6R, 9R, 10R, 12R, 12aR)-3, 6, 9-trimethyldecahydro-12h-3, 12-epoxy [1, 2] dioxopyran [4, 3-i] isobenzopyran-10-yl ((3, 5, 6-trimethylpyrazine-2-yl) methyl) succinate

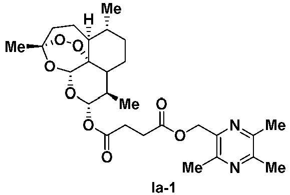

### Step 1. Preparation of intermediate Ia-1-2: 2, 3, 5, 6-tetramethylpyrazine-1-oxide

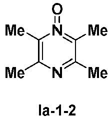

Acetic acid AcOH (20 mL) was slowly added to 2, 3, 5, 6-tetramethylpyrazine (6.86 g, 50 mmol) at 0°C. The temperature of the resulting reaction solution was raised to a room temperature, and H₂O₂ (30%, 20 mL) was added. The resulting mixture was heated up to 50-60°C, with H₂O₂ (30%, 20 mL) being continued to be added, stirred for 16 h, and cooled to the room temperature after a reaction was completed; and then, H₂O (50 mL) was added. The resulting mixture was concentrated to about 10% of its original volume, 50 mL of H₂O was then added, and this operation was repeated for three times. A saturated potassium carbonate aqueous solution was added until a pH value of the mixture reached 9, and then the mixture was extracted with dichloromethane for 4 times. Organic phases were combined, washed with a saturated saline solution, dried with anhydrous MgSO₄, and then concentrated to obtain 6.20 g of a target white crystalline solid compound with a yield of 82%.

### Step 2. Preparation of intermediate Ia-1-3: 3, 5, 6-trimethylpyrazine-2-methyl alcohol acetate

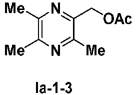

Excess Ac₂O (20 mL) was added to 2, 3, 5, 6-tetramethylpyrazine-1-oxide (3.01 g, 19.7 mmol) to enable a reaction at 100°C for 16 h. After the reaction, the product was cooled to a room temperature, the reaction solution was poured into ice water, and a pH value of the reaction solution was adjusted to 9 with K₂CO₃. Extraction was performed with Et₂O for three times, organic phases were combined, washed with a saturated saline solution, dried with anhydrous MgSO₄, and concentrated under a reduced pressure to obtain a crude product, and the crude product was separated by silica gel chromatography and eluted with 60% ethyl acetate/petroleum ether to obtain 2.36 g of a colorless oily product with a yield of 61%.

### Step 3. Preparation of intermediate Ia-1-4: (3,5,6-trimethylpyrazine-2-yl) methanol

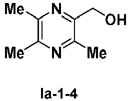

5 M NaOH (10 mL, 50 mmol) was added to 2.4 g of the prepared intermediate Ia-1-3, and stirred at a room temperature for 12 h. The resulting reaction solution was extracted with dichloromethane for 3 times. Organic phases were combined, washed with a saturated saline solution, dried with MgSO₄, and concentrated to obtain a colorless oily product Ia-1-4 with a yield of 100%.

### Step 4. Preparation of target product Ia-1: (3R, 5aS, 6R, 9R, 10R, 12R, 12aR)-3, 6, 9-trimethyldecahydro-12h-3, 12-epoxy [1, 2] dioxopyran [4, 3-i] isobenzopyran-10-yl ((3, 5, 6-trimethylpyrazine-2-yl) methyl) succinate

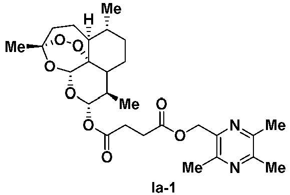

Intermediates Ia-1-4 (0.242 g) and Ia-1-5 (1.153 g), 1-ethyl-(3-dimethylaminopropyl) carbodiimide hydrochloride (0.592 g) and 1-hydroxybenzotriazole (0.183 g) were dissolved in 20 mL of dichloromethane and stirred at a room temperature for 2 h. After the reaction was completed, the resulting reaction solution was washed with 20 mL of water and a saturated saline solution in turn, dried with MgSO₄, and concentrated to obtain a pale yellow crystalline solid crude product. The crude product was separated by silica gel column chromatography and eluted with 50% ethyl acetate/petroleum ether to obtain 0.76 g of colorless crystalline solid with a yield of 97%.

### Embodiment 2. Preparation of compound Ia-2:

(3R, 5aS, 6R, 9R, 10R, 12R, 12aR)-3, 6, 9-trimethyldecahydro-12h-3, 12-epoxy [1, 2] dioxopyran [4, 3-i] isobenzopyran-10-yl ((3, 5, 6-trimethylpyrazine-2-yl) methyl) malonate

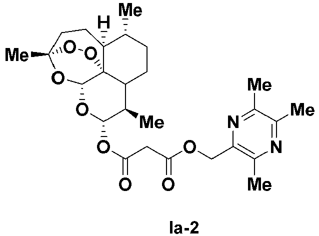

### Step 1: Preparation of intermediate Ia-2-6: dihydroartemisinin-10-α-malonate monoester

Intermediate Ia-1-5 (0.284 g) and N, N-dimethylaminopyridine (0.122 g) were dissolved in 10 mL of dichloromethane, malonic anhydride (0.090 g) was added, and the resulting mixture was stirred at a room temperature for 6 h. After the reaction was complete, the resulting reaction solution was washed with 20 mL of water and saturated sodium chloride in turn, dried with MgSO₄, and concentrated to obtain 0.326 g of pale yellow crystalline solid. After recrystallization, 0.314 g of a pure product was obtained with a yield of 85%.

Intermediate Ia-1-4 was prepared according to the method of Embodiment 1.

Step 2. Preparation of target product Ia-2: (3R, 5aS, 6R, 9R, 10R, 12R, 12aR)-3, 6, 9-trimethyldecahydro-12h-3, 12-epoxy [1, 2] dioxopyran [4, 3-i] isobenzopyran-10-yl ((3, 5, 6-trimethylpyrazine-2-yl) methyl) malonate

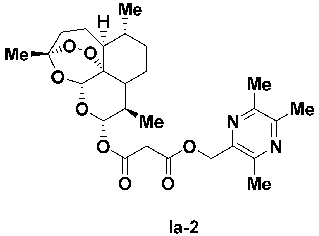

Intermediate Ia-1-4 (0.152 g) and Ia-2-6 (0.370 g), 1-ethyl-(3-dimethylaminopropyl) carbodiimide hydrochloride (0.229 g) and 1-hydroxybenzotriazole (0.162 g) were dissolved in 20 mL of dichloromethane and stirred at a room temperature for 2 h. After the reaction was completed, the resulting reaction solution was washed with 20 mL of water and a saturated saline solution in turn, dried with MgSO₄, and concentrated to obtain a pale yellow crystalline solid crude product. The crude product was separated by silica gel column chromatography and eluted with 50% ethyl acetate/petroleum ether to obtain 0.48 g of colorless crystalline solid with a yield of 95%.

### Embodiment 3. Preparation of compound Ia-3:

(3R, 5aS, 6R, 9R, 10R, 12R, 12aR)-3, 6, 9-trimethyldecahydro-12h-3, 12-epoxy [1, 2] dioxopyran [4, 3-i] isobenzopyran-10-yl ((3, 5, 6-trimethylpyrazine-2-yl) methyl) maleate

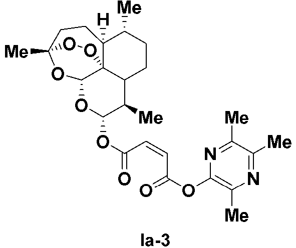

### Step 1: Preparation of intermediate Ia-3-6: dihydroartemisinin-10-α-maleate monoester

Intermediate Ia-1-5 (0.284 g) and N, N-dimethylaminopyridine (0.122 g) were dissolved in 10 mL of dichloromethane, maleic anhydride (0.098 g) was added, and the resulting mixture was stirred at a room temperature for 6 h. After the reaction was complete, the resulting reaction solution was washed with 20 mL of water and saturated sodium chloride in turn, dried with MgSO₄, and concentrated to obtain 0.351 g of pale yellow crystalline solid. After recrystallization, 0.347 g of a pure product was obtained with a yield of 91%.

Intermediate Ia-1-4 was prepared according to the method of Embodiment 1.

### Step 2. Preparation of target product Ia-3: (3R, 5aS, 6R, 9R, 10R, 12R, 12aR)-3, 6, 9-trimethyldecahydro-12h-3, 12-epoxy [1, 2] dioxopyran [4, 3-i] isobenzopyran-10-yl ((3, 5, 6-trimethylpyrazine-2-yl) methyl) maleate

Intermediate Ia-1-4 (0.152 g) and Ia-2-6 (0.382 g), 1-ethyl-(3-dimethylaminopropyl) carbodiimide hydrochloride (0.229 g) and 1-hydroxybenzotriazole (0.162 g) were dissolved in 20 mL of dichloromethane and stirred at a room temperature for 2 h. After the reaction was completed, the resulting reaction solution was washed with 20 mL of water and a saturated saline solution in turn, dried with MgSO₄, and concentrated to obtain a pale yellow crystalline solid crude product. The crude product was separated by silica gel column chromatography and eluted with 50% ethyl acetate/petroleum ether to obtain 0.49 g of colorless crystalline solid with a yield of 98%.

### Embodiment 4. Preparation of compound Ia-4:

(3R, 5aS, 6R, 9R, 10R, 12R, 12aR)-3, 6, 9-trimethyldecahydro-12h-3, 12-epoxy [1, 2] dioxopyran [4, 3-i] isobenzopyran-10-yl ((3, 5, 6-trimethylpyrazine-2-yl) methyl) glutarate

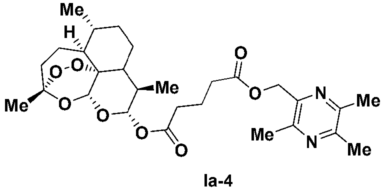

### Step 1: Preparation of intermediate Ia-4-6: dihydroartemisinin-10-α-glutarate monoester

Intermediate Ia-1-5 (0.284 g) and N, N-dimethylaminopyridine (0.122 g) were dissolved in 10 mL of dichloromethane, glutaric anhydride (0.115 g) was added, and the resulting mixture was stirred at a room temperature for 6 h. After the reaction was complete, the resulting reaction solution was washed with 20 mL of water and saturated sodium chloride in turn, dried with MgSO₄, and concentrated to obtain 0.361 g of pale yellow crystalline solid. After recrystallization, 0.346 g of a pure product was obtained with a yield of 87%.

Intermediate Ia-1-4 was prepared according to the method of Embodiment 1.

### Step 2. Preparation of target product Ia-4: (3R, 5aS, 6R, 9R, 10R, 12R, 12aR)-3, 6, 9-trimethyldecahydro-12h-3, 12-epoxy [1, 2] dioxopyran [4, 3-i] isobenzopyran-10-yl ((3, 5, 6-trimethylpyrazine-2-yl) methyl) glutarate

Intermediate Ia-1-4 (0.152 g) and Ia-4-6 (0.398 g), 1-ethyl-(3-dimethylaminopropyl) carbodiimide hydrochloride (0.229 g) and 1-hydroxybenzotriazole (0.162 g) were dissolved in 20 mL of dichloromethane and stirred at a room temperature for 2 h. After the reaction was completed, the resulting reaction solution was washed with 20 mL of water and a saturated saline solution in turn, dried with MgSO₄, and concentrated to obtain a pale yellow crystalline solid crude product. The crude product was separated by silica gel column chromatography and eluted with 50% ethyl acetate/petroleum ether to obtain 0.49 g of colorless crystalline solid with a yield of 92%.

### Embodiment 5. Preparation of compound Ia-5:

(3R, 5aS, 6R, 9R, 10R, 12R, 12aR)-3, 6, 9-trimethyldecahydro-12h-3, 12-epoxy [1, 2] dioxopyran [4, 3-i] isobenzopyran-10-yl ((3, 5, 6-trimethylpyrazine-2-yl) methyl) pimelate

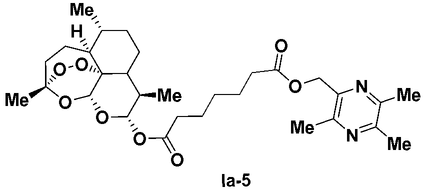

### Step 1: Preparation of intermediate Ia-5-6: dihydroartemisinin-10-α-pimelate monoester

Intermediate Ia-1-5 (0.284 g) and N, N-dimethylaminopyridine (0.122 g) were dissolved in 10 mL of dichloromethane, heptanedioic anhydride (0.142 g) was added, and the resulting mixture was stirred at a room temperature for 6 h. After the reaction was complete, the resulting reaction solution was washed with 20 mL of water and saturated sodium chloride in turn, dried with MgSO₄, and concentrated to obtain 0.384 g of pale yellow crystalline solid. After recrystallization, 0.362 g of a pure product was obtained with a yield of 85%.

Intermediate Ia-1-4 was prepared according to the method of Embodiment 1.

### Step 2. Preparation of target product Ia-5: (3R, 5aS, 6R, 9R, 10R, 12R, 12aR)-3, 6, 9-trimethyldecahydro-12h-3, 12-epoxy [1, 2] dioxopyran [4, 3-i] isobenzopyran-10-yl ((3, 5, 6-trimethylpyrazine-2-yl) methyl) pimelate

Intermediate Ia-1-4 (0.152 g) and Ia-5-6 (0.462 g), 1-ethyl-(3-dimethylaminopropyl) carbodiimide hydrochloride (0.229 g) and 1-hydroxybenzotriazole (0.162 g) were dissolved in 20 mL of dichloromethane and stirred at a room temperature for 2 h. After the reaction was completed, the resulting reaction solution was washed with 20 mL of water and a saturated saline solution in turn, dried with MgSO₄, and concentrated to obtain a pale yellow crystalline solid crude product. The crude product was separated by silica gel column chromatography and eluted with 50% ethyl acetate/petroleum ether to obtain 0.526 g of colorless crystalline solid with a yield of 94%.

### Embodiment 6. Preparation of compound Ia-6:

(3R, 5aS, 6R, 9R, 10R, 12R, 12aR)-3, 6, 9-trimethyldecahydro-12h-3, 12-epoxy [1, 2] dioxopyran [4, 3-i] isobenzopyran-10-yl ((3, 5, 6-trimethylpyrazine-2-yl) methyl) adipate

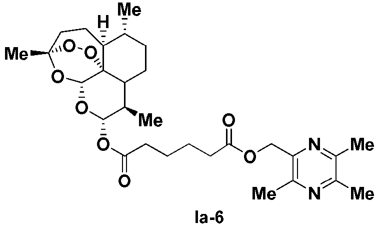

### Step 1: Preparation of intermediate Ia-6-6: dihydroartemisinin-10-α-adipate monoester

Intermediate Ia-1-5 (0.284 g) and N, N-dimethylaminopyridine (0.122 g) were dissolved in 10 mL of dichloromethane, adipic anhydride (0.129 g) was added, and the resulting mixture was stirred at a room temperature for 6 h. After the reaction was complete, the resulting reaction solution was washed with 20 mL of water and saturated sodium chloride in turn, dried with MgSO₄, and concentrated to obtain 0.391 g of pale yellow crystalline solid. After recrystallization, 0.367 g of a pure product was obtained with a yield of 89%.

Intermediate Ia-1-4 was prepared according to the method of Embodiment 1.

### Step 2. Preparation of target product Ia-6: (3R, 5aS, 6R, 9R, 10R, 12R, 12aR)-3, 6, 9-trimethyldecahydro-12h-3, 12-epoxy [1, 2] dioxopyran [4, 3-i] isobenzopyran-10-yl ((3, 5, 6-trimethylpyrazine-2-yl) methyl) adipate

Intermediate Ia-1-4 (0.152 g) and Ia-6-6 (0.412 g), 1-ethyl-(3-dimethylaminopropyl) carbodiimide hydrochloride (0.229 g) and 1-hydroxybenzotriazole (0.162 g) were dissolved in 20 mL of dichloromethane and stirred at a room temperature for 2 h. After the reaction was completed, the resulting reaction solution was washed with 20 mL of water and a saturated saline solution in turn, dried with MgSO₄, and concentrated to obtain a pale yellow crystalline solid crude product. The crude product was separated by silica gel column chromatography and eluted with 50% ethyl acetate/petroleum ether to obtain 0.496 g of colorless crystalline solid with a yield of 91%.

### Embodiment 7. Preparation of compound Ia-7:

(3R, 5aS, 6R, 9R, 10R, 12R, 12aR)-3, 6, 9-trimethyldecahydro-12h-3, 12-epoxy [1, 2] dioxopyran [4, 3-i] isobenzopyran-10-yl ((3, 5, 6-trimethylpyrazine-2-yl) methyl) suberate

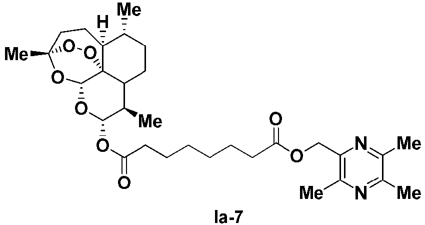

### Step 1: Preparation of intermediate Ia-7-6: dihydroartemisinin-10-α-suberate monoester

Intermediate Ia-1-5 (0.284 g) and N, N-dimethylaminopyridine (0.122 g) were dissolved in 10 mL of dichloromethane, octanedioic anhydride (0.156 g) was added, and the resulting mixture was stirred at a room temperature for 6 h. After the reaction was complete, the resulting reaction solution was washed with 20 mL of water and saturated sodium chloride in turn, dried with MgSO₄, and concentrated to obtain 0.412 g of pale yellow crystalline solid. After recrystallization, 0.387 g of a pure product was obtained with a yield of 88%.

Intermediate Ia-1-4 was prepared according to the method of Embodiment 1.

### Step 2. Preparation of target product Ia-7: (3R, 5aS, 6R, 9R, 10R, 12R, 12aR)-3, 6, 9-trimethyldecahydro-12h-3, 12-epoxy [1, 2] dioxopyran [4, 3-i] isobenzopyran-10-yl ((3, 5, 6-trimethylpyrazine-2-yl) methyl) suberate

Intermediate Ia-1-4 (0.152 g) and Ia-7-6 (0.440 g), 1-ethyl-(3-dimethylaminopropyl) carbodiimide hydrochloride (0.229 g) and 1-hydroxybenzotriazole (0.162 g) were dissolved in 20 mL of dichloromethane and stirred at a room temperature for 2 h. After the reaction was completed, the resulting reaction solution was washed with 20 mL of water and a saturated saline solution in turn, dried with MgSO₄, and concentrated to obtain a pale yellow crystalline solid crude product. The crude product was separated by silica gel column chromatography and eluted with 50% ethyl acetate/petroleum ether to obtain 0.545 g of colorless crystalline solid with a yield of 95%.

### Embodiment 8. Preparation of compound Ia-8:

2, 3, 5-trimethyl-6-[(2-(((3R, 5aS, 6R, 9R, 12R, 12aR)-3, 6, 9-trimethylbicyclo [3.2.1] heptane-12H-3, 12-epoxide [1, 2] dioxacycloheptane [4, 3-i] isochromene-10-yl) oxy) ethoxy) methyl] pyrazine

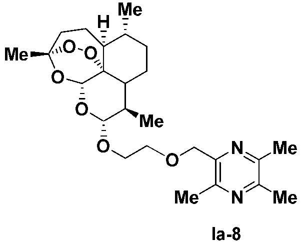

### Step 1: Preparation of intermediate Ia-8-6: dihydroartemisinin-10-α-pimelate monoester

Intermediate Ia-1-5 (0.284 g) and ethylene glycol (0.062 g) were dissolved in 10 mL of dichloromethane, K₂CO₃ was added, and the resulting mixture was stirred at a room temperature for 12 h. After the reaction was complete, the resulting reaction solution was washed with 20 mL of water and saturated sodium chloride in turn, dried with MgSO₄, and concentrated to obtain 0.210 g of white solid. After recrystallization, 0.187 g of a pure product was obtained with a yield of 57%.

Intermediate Ia-1-4 was prepared according to the method of Embodiment 1.

### Step 2. Preparation of target product Ia-8: (3R, 5aS, 6R, 9R, 10R, 12R, 12aR)-3, 6, 9-trimethyldecahydro-12h-3, 12-epoxy [1, 2] dioxopyran [4, 3-i] isobenzopyran-10-yl ((3, 5, 6-trimethylpyrazine-2-yl) methyl) pimelate

Intermediate Ia-1-4 (0.152 g) and Ia-8-6 (0.328 g), and K₂CO₃ (0.229 g) were dissolved in 20 mL of dichloromethane and stirred at a room temperature for 2 h. After the reaction was completed, the resulting reaction solution was washed with 20 mL of water and a saturated saline solution in turn, dried with MgSO₄, and concentrated to obtain a pale yellow crystalline solid crude product. The crude product was separated by silica gel column chromatography and eluted with 50% ethyl acetate/petroleum ether to obtain 0.282 g of colorless crystalline solid with a yield of 61%.

### Embodiment 9. Preparation of compound Ia-9:

(3, 5, 6-trimethylpyrazine-2-yl) methyl 1-(2-oxy-2-(((3R, 5aS, 6R, 9R, 10S, 12R, 12aR)-3, 6, 9-trimethyldecahydro-12H-3, 12-epoxy [1, 2] dioxacycloheptane-10-yl) oxy) ethyl) piperidine-4-formate

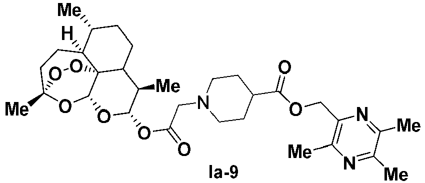

### Step 1: Preparation of intermediate Ia-9-6: dihydroartemisinin-10-α-1-(carboxymethyl) 4-piperidine formate monoester

Intermediate Ia-1-5 (0.284 g), 1-(carboxymethyl) 4-piperidinecarboxylic acid (0.187 g), 1-ethyl-(3-dimethylaminopropyl) carbodiimide hydrochloride (0.229 g), and 1-hydroxybenzotriazole (0.162 g) were dissolved in 20 mL of dichloromethane, dissolved in 10 mL of dichloromethane, and the resulting mixture was stirred at a room temperature for 6 h. After the reaction was complete, the resulting reaction solution was washed with 20 mL of water and saturated sodium chloride in turn, dried with MgSO₄, and concentrated to obtain 0.399 g of pale yellow crystalline solid. After recrystallization, 0.385 g of a pure product was obtained with a yield of 85%.

Intermediate Ia-1-4 was prepared according to the method of Embodiment 1.

### Step 2. Preparation of target product Ia-9: (3R, 5aS, 6R, 9R, 10R, 12R, 12aR)-3, 6, 9-trimethyldecahydro-12h-3, 12-epoxy [1, 2] dioxopyran [4, 3-i] isobenzopyran-10-yl ((3, 5, 6-trimethylpyrazine-2-yl) methyl) suberate

Intermediate Ia-1-4 (0.152 g) and Ia-9-6 (0.453 g), 1-ethyl-(3-dimethylaminopropyl) carbodiimide hydrochloride (0.229 g) and 1-hydroxybenzotriazole (0.162 g) were dissolved in 20 mL of dichloromethane and stirred at a room temperature for 2 h. After the reaction was completed, the resulting reaction solution was washed with 20 mL of water and a saturated saline solution in turn, dried with MgSO₄, and concentrated to obtain a pale yellow crystalline solid crude product. The crude product was separated by silica gel column chromatography and eluted with 50% ethyl acetate/petroleum ether to obtain 0.511 g of colorless crystalline solid with a yield of 87%.

### Embodiment 10. Preparation of compound Ib-1:

(3R, 5aS, 6R, 9R, 10R, 12R, 12aR)-3, 6, 9-trimethyldecahydro-12h-3, 12-epoxy-1, 2-dioxopyran [4, 3-i] isobenzopyran-10-yl ((3, 5, 6-trimethylpyrazine-2-yl) methyl) ether

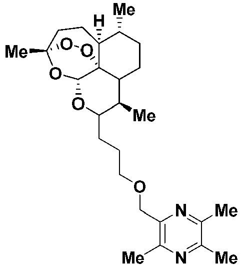

### Step 1:

### Preparation of intermediate Ib-1-1:

Dihydroartemisinin (2 g, 7.034 mmol, 1 equiv) and pyridine (2.78 g, 35.170 mmol, 5 equiv) were dissolved in dimethylformamide (DCM) (20 mL), and benzoyl chloride (1.19 g, 8.441 mmol, 1.2 equiv) was slowly added dropwise at 0°C. After the addition was completed, the reaction mixture was stirred at a room temperature for 16 h. After the reaction was completed, 20 mL of water were added to quench the reaction, the reaction solution was extracted with DCM, organic phases were combined and washed with a saturated NaHCO₃ solution, and the organic phases were dried over anhydrous Na₂SO₄. The solvent was concentrated under reduced pressure and evaporated to dryness to obtain a crude product. The crude product was purified by silica gel column chromatography to obtain 2 g of white crystal intermediate Ib-1-1 with a yield of 73%.

### Preparation of intermediate Ib-1-2:

An intermediate (2 g, 5.149 mmol, 1 equiv) was dissolved in DCM (20 mL), and zinc chloride (0.70 g, 5.149 mmol, 1 equiv) and 4A-MS (1.14 g, 0.676 mmol, 0.13 equiv) were added sequentially at 0°C. After the addition, the reaction mixture was stirred at a room temperature for 16 h. After the reaction was completed, the resulting mixture was diluted with DCM, and washed with a 5% citric acid aqueous solution, a saturated NaHCO₃ aqueous solution and a saline solution. Organic phases were separated, dried with anhydrous Na₂SO₄, concentrated under reduced pressure and evaporated to dryness to obtain a crude product. The crude product was purified by silica gel column chromatography to obtain 1.06 g of colorless oily intermediate Ib-1-2 with a yield of 68%.

### Preparation of intermediate Ib-1-3:

Intermediate Ib-1-2 (900 mg, 2.918 mmol, 1 equiv) was dissolved in 15 mL of tetrahydrofuran (THF), and then cooled to -20°C. 5 mL of a THF solution of borane-dimethyl sulfide complex (265.99 mg, 3.502 mmol, 1.2 equiv, 1M) was slowly added dropwise. Once the dropwise addition was complete, the resulting mixture was heated up to a room temperature and stirred for 2 h. After the reaction was completed, the reaction was quenched with a saturated Na₂CO₃ solution (10 ml), then a 30% H₂O₂ solution (5 ml) was added, and the mixed solution was stirred at a room temperature for 30 min. After the reaction was completed, the solvent was removed under vacuum. The residue was extracted with DCM and washed with water and a saturated saline solution. Organic phases were dried with Na₂SO₄, and the solvent was evaporated to dryness to obtain a crude product. Intermediate Ib-1-3 was 960 mg of brown solid, which was used directly without separation.

### Preparation of compound Ib-1:

Ib-1-3 (960 mg, crude product, 1 equiv) was dissolved in 10 mL of THF, NaH (85 mg, 3.529 mmol, 1.2 equiv) was added at 0°C and stirred for 30 min, intermediate Ib-1-4 (760 mg, 3.529 mmol, 1.2 equiv) was added, and resulting mixture was heated up to a room temperature and stirred for 2 h. After the reaction was completed, water was added to quench the reaction, and the reaction solution was extracted with DCM. Organic phases were combined and washed with water and a saturated saline solution, and the solvent was concentrated under reduced pressure and evaporated to dryness to obtain a crude product. The crude product was purified by silica gel column chromatography to obtain 720 mg of a colorless oily compound Ib-1, with a yield of 53% in total with the previous step.

Embodiment 11. The beneficial effects of the present invention will be further illustrated below through experimental data.

Experimental study on the antimalarial activity of Artesunazine (Example Ia-1) in vitro and vivo.
I. Protection of Artesunazine on mice infected by *Plasmodium berghei* ANKA strain.
(I) Experimental materials:
1. Animals: C57BL/6J, weighing 16-18 g, 10 in each group, 50 in total.
2. Plasmodium strain: *Plasmodium berghei* ANKA strain, donated by Professor Zhao Ya of Air Force Medical University of the People's Liberation Army of China (The Fourth Military Medical University), was preserved in our laboratory by blood transmission or freezing.
3. Drugs and preparation methods: Artesunazine and a positive control drug artesunate were dissolved in a mixed solvent of polyethylene glycol 400 (PEG400) and water (PEG400:water=4:1), serving as a preparation for immediate use.

(II) Experimental methods
1. Plasmodium resuscitation and passage: A *Plasmodium berghei* ANKA strain frozen in liquid nitrogen was taken out and placed in a water bath kettle at 37°C. After melting, it was immediately intraperitoneally inoculated into C57BL/6J mice (0.2 mL/mouse). When the infection rate of the breeding mice reached 15-30%, their eyeballs were removed and blood was collected in an anticoagulant tube. After shaking well, the blood was transmitted to offspring C57BL/6J mice with a 1 mL syringe at an injection dose of 0.2 mL/mouse.
2. Plasmodium inoculation: C57BL/6J mice were weighed and fed adaptively. When the infection rate of offspring C57BL/6J mice reached 15-30%, the total number of red blood cells and the number of pRBCs were counted after their eyeballs were removed and blood was collected. The blood was injected intraperitoneally into model group C57BL/6J mice at an injection dose of 0.2 mL (including 1×10⁷ pRBCs) per mouse, which was the 0^{th} day (D0) of inoculation.
3. Animal grouping: After inoculation, the experimental mice were randomly divided into 5 groups, including a model (M) group, high, medium and low dose Artesunazine groups (i.e., 40 mg/kg, 20 mg/kg, and 10 mg/kg Artesunazine), and an artesunate group (i.e., 15.6 mg/kg AS, clinically equivalent dose). The dosage of the medium dose Artesunazine group (20mg/kg Artesunazine) is equivalent to that of the artesunate group (15.6 mg/kg). There are 10 mice in each group, a total of 50 mice.
4. Determination of survival rate
   From the 0^{th} day of inoculation, the death of mice was observed every day for 28 consecutive days. $Survival rate = \frac{Number of surviving mice}{Number of experimental mice} \times 100 %$
5. Determination of recurrence rate
   After inoculation, the mice were randomly divided into groups and administered via nasal drip for 3 h. After four consecutive days of administration, blood smear test began at D4. A small drop of blood was taken from the tail tip of a mouse and dropped onto a glass slide. The blood drop was pushed to one end at a certain angle with the edge of another glass slide to make a thin blood film with appropriate thickness. After the thin blood film was air-dried, it was fixed with methanol and placed until completely air-dried. After a Giemsa stain solution was diluted at a ratio of 1:9 for staining (completely covering the fixed thin blood film) for 20 min, the thin blood film was rinsed under a faucet, and the water on the surface of the film was quickly absorbed with filter paper. A drop of cedar oil was dropped onto a glass slide for examination under a 100x oil microscope to count the number of parasitized red blood cells contained in 1000 red blood cells. The mouse recurrence rate was calculated by the ratio of the number of mice with recurrence to the total number of mice. $Recurrence rate = \frac{Number of mice with recurrence}{Number of experimental mice} \times 100 %$
6. RMCBS score determination
   A rapid mouse coma behavior scale (RMCBS) was used to evaluate the neurological function of experimental mice. Starting from D0, the mice were scored in 10 aspects: gait, balance, motion-related behavior, body position, limb strength, touch response, auricle reflex, toe touch response, offensive response, offensive behavior, and health behavior. Normal mice had smooth fur and stable gait, stretched their bodies when walking, could explore four corners of a grid within 15 s, and exhibited obvious exploratory behaviors, touch behaviors, auricle response, toe response, touch reflex, and the like. However, model mice had less exploratory behaviors within 120 s, were messy in fur, had ataxia, and arched backs. When stimulating model mice, their reflexes decreased or disappeared. Scoring was based on the performance of the mice, with each item scoring 0-2 points, for a total of 20 points. At a fixed time every day, the mice were tested in a fixed order. During testing, the mice were placed in a box with checkered paper (31.8 cm×19.8 cm×10.5 cm in length, width, and height) on the bottom from a top left corner, and touched with a 3 mm diameter long rod. The test time for one mouse was about 3 min, and the imprint left by the previous mouse needed to be wiped off after each test.
7. Temperature measurement
   The anal temperature of mice was measured starting from D0 after inoculation, and the observation ended on the 14^{th} day.
8. Body weight measurement
   The body weigh of mice was measured starting from D0 after inoculation, and the observation ended on the 14^{th} day.

(III) Experimental results
1. Effect of Artesunazine on survival rate of cerebral malaria model mice
The survival rates of all groups of mice were compared after 28 days of observation since administration was completed. The results showed that all the mice in the M (model) group died on the 15^{th} day, and the survival time of the mice with cerebral malaria was significantly prolonged in all the administration groups. The survival rate of the mice in the high dose Artesunazine (40 mg/kg) group was 100%, the survival rate of the mice in the medium dose Artesunazine (20 mg/kg) group was 90%, the survival rate of the mice in the low dose Artesunazine (10 mg/kg) group was 50%, and the survival rate of the mice in the artesunate (AS) group was 40%. The therapeutic effect of the high and medium dose Artesunazine groups on the mice with cerebral malaria was better than that of the artesunate group (P<0.001).
2. Effect of Artesunazine on recurrence of cerebral malaria model mice
The recurrence rates of all groups of mice were compared after 28 days of observation since administration was completed. The results showed that the infection rate of the model group was 100% on the 4^{th} day. After 4 days of administration, the plasmodia were completely killed in the 40 mg/kg Artesunazine group and the 20 mg/kg Artesunazine group, and 10% of the 10 mg/kg Artesunazine group and the AS group experienced recurrence. On the 7^{th} day, there was no recurrence in the 40 mg/kg Artesunazine group and the 20 mg/kg Artesunazine group, and the recurrence rates in the 10 mg/kg Artesunazine group and the AS group were 80% and 90% respectively. After D28, the recurrence rates of the 40 mg/kg Artesunazine group and the 20 mg/kg Artesunazine group were 10%, and the recurrence rates of the 10 mg/kg Artesunazine group and the AS group were 100%. The inhibition effect of the high and medium dose Artesunazine groups on plasmodium recurrence was better than that of the AS group.

**Table 1 Effects of drugs on recurrence of cerebral malaria model mice**

| Group | Recurrence rate (%) | | |
|---|---|---|---|
| | D4 | D7 | D28 |
| 10 mg/kg Artesunazine | 10 | 80 | 100 |
| 20 mg/kg Artesunazine | 0 | 0 | 10 |
| 40 mg/kg Artesunazine | 0 | 0 | 10 |
| AS | 10 | 90 | 100 |

3. Effect of Artesunazine on neurological function of cerebral malaria model mice (RMCBS score)
The experimental observation was carried out by an RMCBS scoring method, i.e., a classical neurological function evaluation method of cerebral malaria. The results showed that the RMCBS score of the model group began to decrease gradually on the 6^{th} day after inoculation, and at the end point of observation, the score decreased to 14.00±0.26. During the observation period, there was no decrease in the RMCBS scores of the 40 mg/kg Artesunazine group and the 20 mg/kg Artesunazine group, both of which remained stable at around 20 points. The RMCBS scores of the AS group and the 10 mg/kg Artesunazine group decreased gradually on the 9^{th} and 10^{th} day after inoculation, and at the end point of observation, the scores decreased to 16.22±0.55 and 18.40±0.48, respectively. By the end of observation, the RMCBS scores of all the dose groups were significantly higher than that of the model group (P<0.001), and the RMCBS score of the AS group was significantly higher than that of the model group (*P*<0.01). The RMCBS scores of the high, middle and low dose Artesunazine groups were significantly higher than that of the AS group (*P*<0.001, *P<*0.001, *P<*0.01)*.* The high, middle and low dose Artesunazine groups and the AS group were able to effectively ameliorate the pathological changes of behavior and nerves in mice with malaria, such as reduced desire to explore in the late stage, messy fur, ataxia, back arching, limb weakness, and reduced or absent reflexes. Moreover, the overall ethological improvement of the mice in all the dose Artesunazine groups was better than that in the AS group.
4. Effect of Artesunazine on temperature of cerebral malaria model mice
During the observation period, the temperature of the mice in the model group decreased to 36.82±0.42 at the end of observation, and the temperature of the mice in the 10 mg/kg Artesunazine group, the 20 mg/kg Artesunazine group, the 40 mg/kg Artesunazine group and the AS group decreased significantly (*P*<0.001, *P<*0.001, *P<*0.001, *P<*0.001)*.* All the administration groups could obviously inhibit the abnormal decrease of the temperature of the cerebral malaria model mice, and the 20 mg/kg Artesunazine group and the 40 mg/kg Artesunazine group better maintained the temperature of the mice with cerebral malaria compared with the AS group (P<0.001, P<0.001).
5. Effect of Artesunazine on body weight of cerebral malaria model mice
During the first 6 days of the observation period, the body weight of the mice in all the groups showed a gradually increasing trend, and the degree of weight gain of the mice in all the groups was basically the same. On the 6^{th} day, the body weight of the mice in the model group began to decrease, and at the end of observation, their body weight decreased to 13.15±0.29. The body weight of the mice in the 10 mg/kg Artesunazine group and the AS group gradually decreased from the 7^{th} day and 9^{th} day after inoculation, and at the end of the observation, their body weight decreased to 16.66±0.45 and 15.41±0.50, respectively. There was no significant decrease in the body weight of the mice in the 40 mg/kg Artesunazine group and the 20 mg/kg Artesunazine group throughout the observation period. All the administration groups were able to significantly relieve weight loss in the mice with cerebral malaria (*P<*0.001*, P<*0.001, *P<*0.001, *P<*0.01)*,* and the relieving effect of the 40 mg/kg Artesunazine group and the 20 mg/kg Artesunazine group on the weight loss in the mice with cerebral malaria was better than that of the AS group (*P<*0.001, *P<*0.001)*.*
II. Pharmacodynamic study on the treatment of Plasmodium falciparum cultured in vitro by Artesunazine

### (I) Experimental materials

Experimental drugs and reagents: bovine serum albumin complete medium for Plasmodium falciparum (see medium preparation for details). 0.16% saponin (Sigma, LOT: BCBR4223V): prepared immediately by adding 10 mL of deionized water to 0.016 g of saponin, and mixing well. 0.1M EDTA (Sigma, LOT: BCBR3854V):

prepared by adding 20 mL of deionized water to 0.5844g of EDTA (MW: 292.24 g/mol), and mixing well. 0.4M Tris-Base (Sigma, LOT: SLBR9609V): prepared by adding 20 mL of deionized water to 0.969g of Tris-Base (MW=121.14 g/mol), and mixing well. 0.8% Triton X-100 (Sigma, LOT: SLBR3411V):

prepared by adding 9.92 mL of deionized water to 80 µL of liquid Triton X-100, and mixing well. Lysis buffer: prepared by taking 2 ml of each of 0.16% saponin, 0.1 M EDTA, 0.4 M Tris-Base, 0.8% Triton X-100 and deionized water, as well as 10 µL of SYBR Green I (Invitrogen, LOT: 1797921), and mixing well. Experimental equipment: incubator, fluorescent enzyme-linked immunosorbent assay reader, biosafety cabinet, centrifuge, drying oven, microscope, and pressure cooker. Experimental consumables: pipettes (1000, 200, 100, 10, 2.5 µL) and pipette tips, 15 mL centrifuge tubes, glass slides, and culture dishes

### (II) Experimental methods

Plasmodium falciparum in the ring stage was synchronized. Smearing was carried out and the infection rate was counted. The synchronized parasitized red blood cells, healthy red blood cells and mediums were matched in a reasonable ratio, resulting in an in vitro culture system of plasmodia (ring bodies, 2 to 4 hours-old) with a hematocrit of 2% and an infection rate of 1%. A serum-free cell cryopreservation medium (RPMI) 1640 was laid with a plate (50 µL/well). A1-A10 was paved with 25 µl of drugs (with 8 complex wells for each drug) and mixed evenly, and then diluted in multiple proportions (Ax-Hx). Except for A12-H12, 50 µL of a plasmodium culture solution was spread in all the other wells; and A12-H12 was spread with 50 µl of healthy red blood cells (in the medium with a hematocrit of 2%). Plasmodium falciparum was cultured for 72 h under the conventional in vitro culture conditions (5% O₂, 3% CO₂, 92% N₂, 37°C). 100 µl of a lysis buffer was added to each of the wells and put into a drawer to avoid light for 1 h. Preparation of a lysis buffer (final concentration): 40 mM Tris with a pH of 7.5, 10 mM EDTA, 0.016% saponin (fresh), 0.08% Triton X-100, and Sybr Green I (invitrogen 10000×) were diluted 1000 times. Determination by enzyme-linked immunosorbent assay reader: Ex=485 nm; Em=525 nm.

In this experiment, SYBR Green assay was used to determine the median inhibitory concentration (IC50) value of the drug for treating Plasmodium falciparum 3D7. Nonlinear regression was used to calculate the value by means of software graphpad prism 8.0 (the value was equal to average value plus or minus standard error), and a four-parameter logistic fitting curve was used. Each value in the curve was equal to average value plus or minus standard error in eight biological repetitive experiments.

### (III) Experimental results

### (1) Efficacy analysis of compound Ia-1 in treatment of human Plasmodium falciparum standard strain 3D7

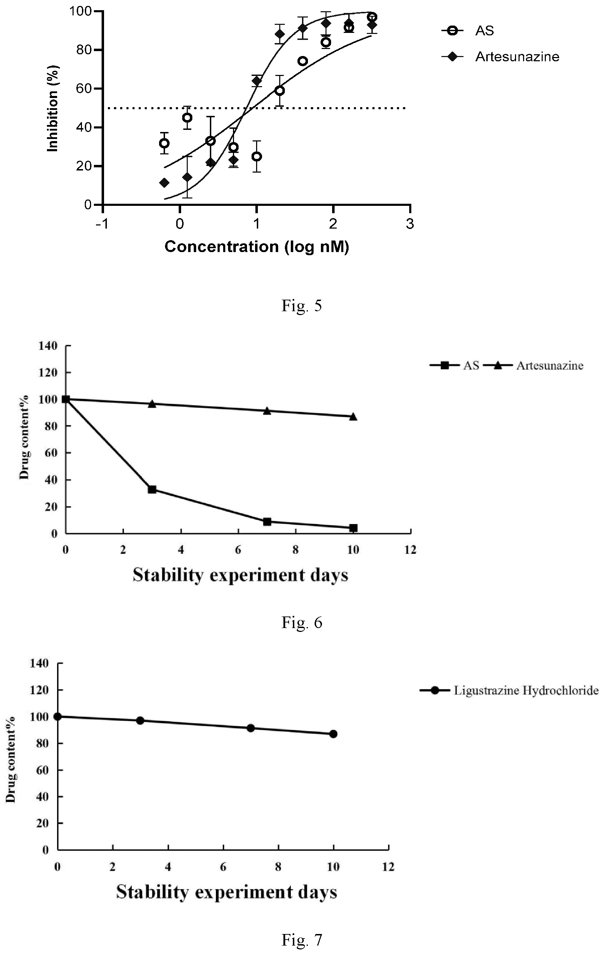

Compound Ia-1 exhibits strong inhibitory effect on parasite proliferation within a nanomolar concentration range (IC50=7.358 nM), indicating that although the compound is comparable in efficacy to standard antimalarial drugs such as chloroquine (approximately 2.360 nM), mefloquine (approximately 3.962 nM), and artemisinin (approximately 11.768 nm), it has a strong inhibitory effect on plasmodia. Furthermore, the control drug artesunate has a significant inhibitory effect on the proliferation of Plasmodium falciparum 3D7, with an IC50 value of 8.825 nM (as shown in Fig. 5).

### (2) Efficacy analysis of compound Ia-2 in treatment of human Plasmodium falciparum standard strain 3D7

Compound Ia-2 has a significant inhibitory effect on parasite proliferation within a nanomolar concentration range (IC50=12.52 nM).

### (3) Efficacy analysis of compound Ia-3 in treatment of human Plasmodium falciparum standard strain 3D7

Compound Ia-3 has a significant inhibitory effect on the proliferation of Plasmodium falciparum 3D7 within a nanomolar concentration range (IC50=6.863 nM).

### (4) Efficacy analysis of compound Ia-4 in treatment of human Plasmodium falciparum standard strain 3D7

Compound Ia-4 has a significant inhibitory effect on the proliferation of Plasmodium falciparum 3D7 within a nanomolar concentration range (IC50=33.92 nM).

### (5) Efficacy analysis of compound Ia-5 in treatment of human Plasmodium falciparum standard strain 3D7

Compound Ia-5 has a significant inhibitory effect on the proliferation of Plasmodium falciparum 3D7 within a nanomolar concentration range (IC50=2.953 nM).

### (6) Efficacy analysis of compound Ia-6 in treatment of human Plasmodium falciparum standard strain 3D7

Compound Ia-6 has a significant inhibitory effect on the proliferation of Plasmodium falciparum 3D7 within a nanomolar concentration range (IC50=14.29 nM).

### (7) Efficacy analysis of compound Ia-7 in treatment of human Plasmodium falciparum standard strain 3D7

Compound Ia-7 has a significant inhibitory effect on the proliferation of Plasmodium falciparum 3D7 within a nanomolar concentration range (IC50=50.68 nM).

### (8) Efficacy analysis of compound Ia-8 in treatment of human Plasmodium falciparum standard strain 3D7

Compound Ia-8 has a significant inhibitory effect on the proliferation of Plasmodium falciparum 3D7 within a nanomolar concentration range (IC50=107.2 nM).

### (9) Efficacy analysis of compound Ia-9 in treatment of human Plasmodium falciparum standard strain 3D7

Compound Ia-9 has a significant inhibitory effect on the proliferation of Plasmodium falciparum 3D7 within a nanomolar concentration range (IC50=32.73 nM).

In summary, Compounds Ia-2 to Ia-9 have significant inhibitory effects on the proliferation of Plasmodium falciparum 3D7 within the nanomolar concentration range, and their efficacy is comparable to that of Compound Ia-1.

### III. Study on stability of Artesunazine

### (I) Experimental method

### 1. Study on stability of Artesunazine

160.2 mg of Artesunazine was weighed and put into a penicillin bottle, 8 mL of a 80% polyethylene glycol 400 solution was added, and the Artesunazine was completely dissolved by ultrasonic treatment, resulting in about 20.25 mg/mL Artesunazine solution. Subsequently, the penicillin bottle filled with the solution was sealed, covered with aluminum foil paper for light avoidance treatment, and placed in a stability test box at 40°C; and 0.5 mL to 25 mL of the solution was sampled into volumetric flasks on days 0, 3, 7, and 10, respectively. After diluting with methanol and shaking well, the content of the Artesunazine was determined by an ultra performance liquid chromatography (UPLC) method.

The UPLC determination conditions for the Artesunazine are as follows: Instrument: ultra performance liquid chromatography Waters ACQUITY UPLC, chromatographic column: ACQUITY UPLC HSS T3 Column (100 Å, 1.8 µm, 2.1 mm×100 mm), detection wavelength: 295 nm, mobile phase: acetonitrile-0.1% phosphoric acid water (35:65), flow rate: 0.3 ml/min, column temperature: 30°C, sample room temperature: 25°C, injection volume: 3 µL.

### 2. Study on the stability of artesunate and ligustrazine hydrochloride in the same solution

A mixed solution of artesunate and ligustrazine hydrochloride was prepared according to a molar concentration of 20 mg/mL Artesunazine, specifically as follows: 148.5 mg of artesunate and 66.2 mg of ligustrazine hydrochloride were weighed and put into the same penicillin bottle, 10mL of a 80% polyethylene glycol 400 aqueous solution was added, and ultrasonic treatment was performed until complete dissolution to obtain a mixed solution of artesunate and ligustrazine hydrochloride with a concentration of 14.85 mg/mL; the penicillin bottle filled with the solution was sealed, covered with aluminum foil paper for light avoidance treatment, and placed in a stability test box at 40°C; and 0.5 mL to 25 mL of the solution was sampled into volumetric flasks on days 0, 3, 7, and 10, respectively. After diluting with methanol and shaking well, the content of the Artesunazine was determined by a UPLC method.
(1) The UPLC determination conditions for artesunate are as follows: Instrument: ultra performance liquid chromatography Waters ACQUITY UPLC, chromatographic column: ACQUITY UPLC HSS T3 Column (100 Å, 1.8 µm, 2.1 mm×100 mm), detection wavelength: 216 nm, mobile phase: acetonitrile-0.1% phosphoric acid water (44:56), flow rate: 0.3 ml/min, column temperature: 30°C, sample room temperature: 25°C, injection volume: 4 µL.
(2) The UPLC determination conditions for ligustrazine hydrochloride are as follows: Instrument: ultra performance liquid chromatography Waters ACQUITY UPLC, chromatographic column: ACQUITY UPLC HSS T3 Column (100 Å, 1.8 µm, 2.1 mm×100 mm), detection wavelength: 295 nm, mobile phase: acetonitrile-0.1% phosphoric acid water (10:90), flow rate: 0.3 mL/min, column temperature: 30°C, sample room temperature: 25°C, injection volume: 1 µL.

### (II) Experimental results

The stability of the artesunate-ligustrazine hydrochloride compound in the 80% polyethylene glycol 400 solution was poor. The content of artesunate rapidly decreased in a dark environment at 40°C, and the color of the mixture solution gradually turned yellow. Compared with this compound, the stability of the Artesunazine in the 80% polyethylene glycol 400 solution was significantly improved, and the color of the Artesunazine solution was basically unchanged. This result provides a good foundation for the development of novel antimalarial drugs (Fig. 6 and Fig. 7).

## Claims

1. A drug comprising Formula I or a pharmaceutically acceptable salt thereof: 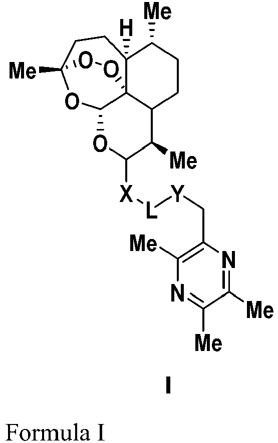
wherein X and Y are each independently selected from the group consisting of CH₂, O, S, and NH; and
L is a linker selected from the group consisting of: 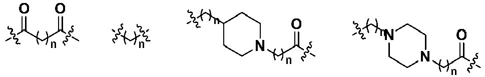 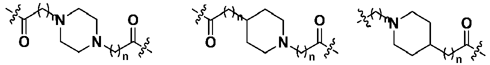
wherein n is an integer from 0 to 6.

2. The drug or the pharmaceutically acceptable salt thereof according to claim 1, comprising a structure: 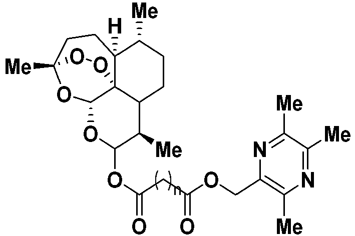 wherein n is an integer from 0 to 6.

3. The drug or the pharmaceutically acceptable salt thereof according to claim 1, comprising a structure:

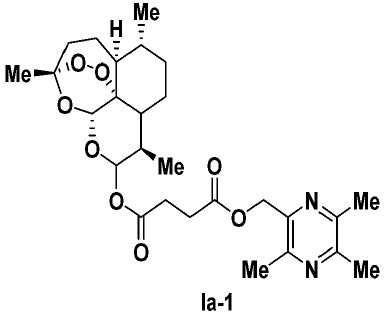

4. The drug or the pharmaceutically acceptable salt thereof according to any one of claims 1 to 3, wherein the pharmaceutically acceptable salt is an addition salt formed with an acid selected from the group consisting of hydrochloric acid, hydrobromic acid, sulfuric acid, phosphoric acid, methanesulfonic acid, ethanesulfonic acid, p-toluenesulfonic acid, benzenesulfonic acid, naphthalene disulfonic acid, acetic acid, propionic acid, lactic acid, trifluoroacetic acid, maleic acid, citric acid, fumaric acid, oxalic acid, tartaric acid, benzoic acid, and the like; and hydrochloric acid, hydrobromic acid, sulfuric acid, lactic acid, pyruvic acid, acetic acid, trifluoroacetic acid, maleic acid, benzenesulfonic acid, and succinic acid.

5. A pharmaceutical composition, comprising the drug or the pharmaceutically acceptable salt thereof according to any one of claims 1 to 4.

6. The pharmaceutical composition according to claim 5, wherein the pharmaceutical composition is in the form of a pharmaceutical preparation and further comprises pharmaceutically acceptable excipients as required for the preparation.

7. The pharmaceutical composition according to claim 5, selected from any pharmaceutical dosage forms for administration.

8. The pharmaceutical composition according to claim 7, selected from a nasal preparation, an injection, or an oral preparation.

9. A preparation method of the drug or the pharmaceutically acceptable salt thereof according to claim 2, comprising following steps: 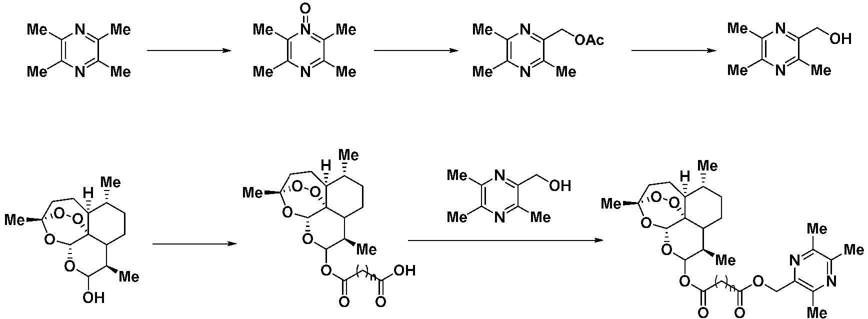 or 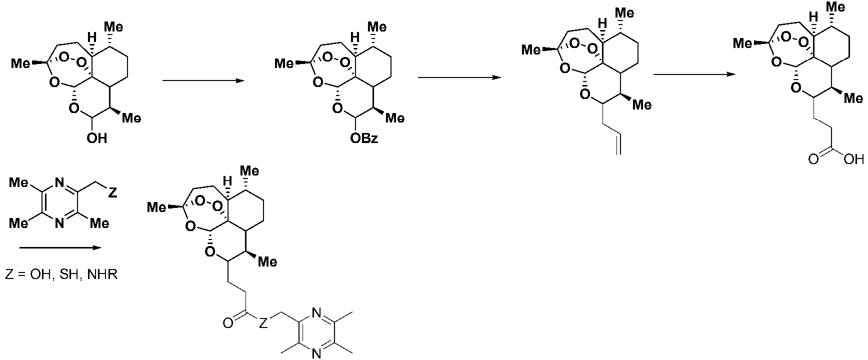 or

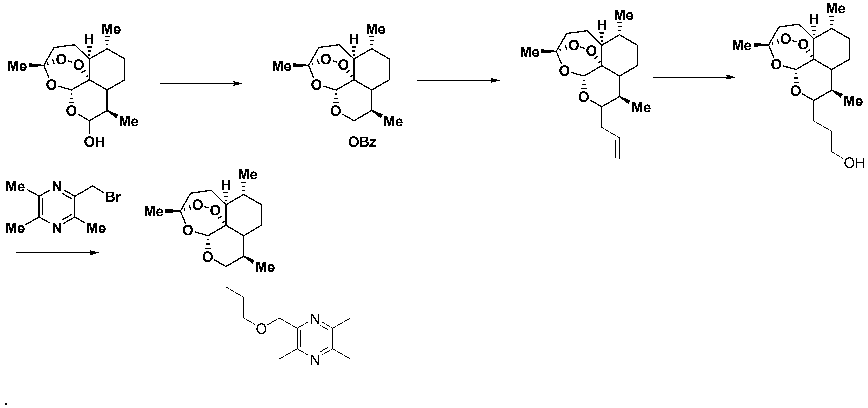

10. Use of the drug or the pharmaceutically acceptable salt thereof according to claim 1 in the preparation of a drug for treating or preventing severe malaria.
